# EUROPÄISCHE PATENTANMELDUNG

(11) **EP 3 793 334 A1**
(43) Veröffentlichungstag der Anmeldung: **17.03.2021**
(21) Anmeldenummer: 20195225.6
(22) Anmeldetag: 09.09.2020
(51) Int. Cl.: H05H 1/24, A61N 1/44

(54) **VORRICHTUNG UND VERFAHREN ZUR HAUT- UND INSBESONDERE WUNDBEHANDLUNG UNTER VERWENDUNG VON PLASMA**

(30) Priorität: 16.09.2019 DE 102019006536
(71) Anmelder: Ushio Germany GmbH, 85643 Steinhöring (DE)
(72) Erfinder: KAISER, Dr.,, Mathias, 85643 Steinhöring (DE)
(74) Vertreter: Tomerius, Isabel

(57) **Zusammenfassung**

Die Erfindung betrifft eine Vorrichtung zur Haut- und insbesondere Wundbehandlung unter Verwendung eines Atmosphärendruck-Plasmas, welche einen Plasmagenerator aufweist, der wenigstens eine Elektrode, wenigstens ein Dielektrikum, eine elektrische Versorgungseinheit sowie eine Steuereinheit umfasst und ausgebildet ist, das Atmosphärendruck-Plasma mittels einer dielektrischen Barriereentladung zu erzeugen. Die Steuereinheit ist ausgebildet, den Plasmagenerator in unterschiedlichen Betriebsmodi derart zu betreiben, dass der wenigstens einen Elektrode in einem ersten Betriebsmodus eine erste Anregungsenergie zugeführt wird, die ausreicht, ein Stickstoffmonoxid (NO)-haltiges Plasma zu erzeugen, und in einem zweiten Betriebsmodus eine zweite Anregungsenergie, die ausreicht, ein Ozon (O₃)-haltiges Plasma zu erzeugen. Die erste Anregungsenergie ist dabei größer als die zweite Anregungsenergie.

Die Erfindung betrifft weiterhin ein Verfahren zur Behandlung der Haut und insbesondere einer Wunde unter Verwendung der vorstehend beschriebenen Vorrichtung. Das Verfahren ist gekennzeichnet durch die folgenden Schritte: Auswählen eines der Betriebsmodi aus dem ersten und dem zweiten Betriebsmodus und Steuern der Versorgungseinheit durch die Steuereinheit entsprechend dem ausgewählten Betriebsmodus, sodass der wenigstens einen Elektrode die erste Anregungsenergie oder die zweite Anregungsenergie zugeführt wird.

## Beschreibung

Die Erfindung betrifft eine Vorrichtung und ein Verfahren zur Haut- und insbesondere Wundbehandlung unter Verwendung von Plasma, insbesondere eines kalten Atmosphärendruck-Plasmas.

Es ist bekannt, dass sich die Wundheilung durch Zufuhr bestimmter Gase beschleunigen lässt. Hauptsächlich wird als Gas Stickstoffmonoxid (NO) eingesetzt. Stickstoffmonoxid ist ein körpereigenes Signalmolekül, das im Körper Wundheilungsprozesse auslöst und fördert. Bei der Behandlung wird es der Wunde lokal in geringen Dosen zugeführt und dazu meist entweder chemisch erzeugt (US 9,445,996 B2) oder aus einer Gaskartusche appliziert (US 2007/0065473 A1, WO 2011/082375 A2). In letzterer Druckschrift ist für die Behandlung der Wunde mit Stickstoffmonoxid ein Wundpflaster beschrieben, das auf die Wunde aufgeklebt wird und ein Gasreservoir zur Aufnahme des Stickstoffmonoxids enthält, um dieses im Bereich der Wunde zu konzentrieren und eine längerfristige Behandlung zu ermöglichen.

Weiterhin ist auch bekannt, Stickstoffmonoxid für die Wundbehandlung mithilfe einer elektrisch behinderten Barriereentladung zu erzeugen (WO 2015/088948 A1, WO 2016/192997 A1, WO 2017/158396 A1, WO 2018/093261 A1, WO 2018/127255 A1, WO 2018/189174 A1). Unter einer dielektrischen Barriereentladung (DBD = dielectric barrier discharge) wird eine Entladung verstanden, die zwischen Elektroden gezündet wird, die durch wenigstens ein Dielektrikum getrennt sind. Das Dielektrikum verhindert den Übergang zu einer thermischen Bogenentladung. Es entstehen daher "kalte" Plasmen mit Atmosphärendruck, deren Gastemperatur ungefähr der Raumtemperatur entspricht. In einer Variante kann es sich bei einer der Elektroden um die zu behandelnde Haut handeln. In diesem Fall wird das Plasma unmittelbar zwischen der mit einer Betriebsspannung beaufschlagten Elektrode und der Hautoberfläche gebildet.

Bei der dielektrischen Barriereentladung entstehen neben Stickstoffmonoxid weitere reaktive Spezies wie freie Radikale, Kationen und Elektronen, angeregte Atome und Moleküle sowie Licht, darunter UV-Strahlung. Die Zusammensetzung des Plasmas ist von der Art des Plasmagenerators, der Zusammensetzung des zugeführten Gases sowie der zugeführten Energie abhängig. Zur Herstellung Stickstoffmonoxid-haltigen Plasmas müssen Stickstoff und Sauerstoff enthaltende Gasmischungen eingesetzt werden. Häufig wird Luft als Ausgangsgasmischung zur Erzeugung NO-haltiger Plasmen verwendet. Zu den bei der dielektrischen Barriereentladung neben Stickstoffmonoxid gebildeten reaktiven Spezies zählen unter anderem Sauerstoffradikale, Ozon und andere Stickoxide, insbesondere Stickstoffdioxid. Einige dieser Spezies wie beispielsweise Ozon und Stickstoffdioxid sind in höheren Konzentrationen schädlich, sodass es im Allgemeinen das Ziel des Standes der Technik ist, die Konzentration dieser Verbindungen im der Haut zugeführten Plasma zu verringern. Eine im Stand der Technik beschriebene Schadensbegrenzungsmöglichkeit besteht in einer Begrenzung der Behandlungsdauer zur Vermeidung von Zellschädigungen. Die WO 2013/052548 A2 beschreibt zu diesem Zweck beispielsweise einen Stickstoffdioxid-Filter. Die WO 2010/022871 A1 erwähnt die Verwendung eines UV-Filters. Ozon ist in Stickstoffmonoxid-reichen Plasmen grundsätzlich nicht mehr vorhanden, da dieses mit dem Stickstoffmonoxid unter Bildung von Sauerstoff und Stickstoffdioxid reagiert.

Auf welchen Wirkmechanismen des Plasmas selbst, abgesehen von der Wirkung des Stickstoffmonoxids, die infolge der Plasmabehandlung verbesserte Wundheilung beruht, ist bisher nicht geklärt. Diskutiert werden eine Reduktion der bakteriellen Wundbesiedlung sowie eine Beschleunigung der Wundheilung. Eine repräsentative Meta-Analyse aus dem Jahr 2018 (Assadian, O-jan et. al. "Effects and safety of atmospheric low-temperature plasma on bacterial reduction in chronic wounds and wound size reduction: A systematic review and meta-analysis", Int Wound J (2019) 16, 103-111) kommt zu dem Ergebnis, dass beide Effekte sich am Patienten kaum belegen lassen. Die Feststellung, dass plasmabehandelte Flüssigkeit eine ähnliche Entkeimungswirkung zeigt wie die direkte Plasmabehandlung, stellt viele der bisher postulierten Wirkmechanismen von Plasma infrage. Auf einer ähnlichen Erkenntnis beruht möglicherweise die in der WO 2010/022871 A1 beschriebene Zugabe eines sterilisierenden Additivs zum Plasmagas.

Die bisherigen Untersuchungen zeigen jedoch, dass es wünschenswert wäre, eine Vorrichtung und ein Verfahren zur Haut- und insbesondere Wundbehandlung zur Verfügung zu haben, die sowohl eine Keimreduktion auf der Haut bzw. in der Wunde als auch eine beschleunigte Wundheilung bewirken kann. **Aufgabe** der Erfindung ist es entsprechend, eine solche Vorrichtung und ein derartiges Verfahren anzugeben.

Die Lösung dieser Aufgabe gelingt mit der Vorrichtung gemäß Anspruch 1 sowie dem Verfahren gemäß Anspruch 7. Bevorzugte Ausführungsbeispiele und Verfahrensvarianten sind den jeweiligen Unteransprüchen zu entnehmen.

In einem ersten Aspekt betrifft die Erfindung also eine Vorrichtung zur Haut- und insbesondere Wundbehandlung unter Verwendung eines Atmosphärendruck-Plasmas, welche einen Plasmagenerator aufweist, der wenigstens eine Elektrode, wenigstens ein Dielektrikum, eine elektrische Versorgungseinheit sowie eine Steuereinheit umfasst und ausgebildet ist, das Atmosphärendruck-Plasma mittels einer dielektrischen Barriereentladung zu erzeugen. Die Steuereinheit ist ausgebildet, den Plasmagenerator in unterschiedlichen Betriebsmodi derart zu betreiben, dass der wenigstens einen Elektrode in einem ersten Betriebsmodus eine erste Anregungsenergie zugeführt wird, die ausreicht, ein Stickstoffmonoxid (NO)-haltiges Plasma zu erzeugen, und in einem zweiten Betriebsmodus eine zweite Anregungsenergie, die ausreicht, ein Ozon (O₃)-haltiges Plasma zu erzeugen. Die erste Anregungsenergie ist dabei größer als die zweite Anregungsenergie.

Kernidee der Erfindung ist es also, eine Vorrichtung bereitzustellen, die in unterschiedlichen Betriebsmodi betrieben werden kann, wobei die Betriebsmodi so ausgewählt sind, dass sie zur Bildung unterschiedlich zusammengesetzter Plasmen führen. In einem ersten Betriebsmodus wird dabei mit einer ersten Anregungsenergie ein Plasma gebildet, das Stickstoffmonoxid enthält. In einem zweiten Betriebsmodus wird mit einer zweiten Anregungsenergie, die geringer ist als die erste Anregungsenergie, ein Plasma erzeugt, das Ozon enthält. Unter einem Stickstoffmonoxid-haltigen Plasma soll dabei erfindungsgemäß ein Plasma verstanden werden, das einen Anteil an Stickstoffmonoxid im Plasmagas enthält, wobei der Betriebsmodus zur Erzeugung des Plasmas - hier als erster Betriebsmodus bezeichnet - zweckmäßig dahingehend optimiert ist, dass der Anteil an Stickstoffmonoxid möglichst hoch ist. Da, wie eingangs erwähnt, Stickstoffmonoxid mit Ozon reagiert, ist in einem Plasma mit einem hohen Anteil an Stickstoffmonoxid praktisch kein Ozon vorhanden. Das im ersten Betriebsmodus erzeugte Stickstoffmonoxid-haltige Plasma ist daher im Wesentlichen frei von Ozon und enthält allenfalls noch Spuren dieser Verbindung, die analytisch praktisch nicht mehr nachweisbar sind. Wenn bei der Herstellung eines Plasmas NO-Moleküle entstehen, diese aber sofort mit Ozon reagieren und daher verschwinden, so gilt ein derartiges Plasma nicht als NO-haltiges Plasma. Ein erfindungsgemäßes NO-haltiges Plasma muss auch mit den NO-Molekülen zur Haut- oder Wundbehandlung zur Verfügung stehen, weshalb sich die NO-Moleküle nicht nur in direkter Umgebung der Elektroden befinden dürfen. Im NO-haltigen Plasma befinden sich wenigstens um einen Faktor 10 mehr NO-Moleküle als Ozon-Moleküle, während sich im Ozon-haltigen Plasma wenigstens um einen Faktor 10 mehr Ozon-Moleküle als NO-Moleküle befinden. Dies bezieht sich insbesondere auf das für die Haut- oder Wundbehandlung bereitgestellte Plasma, also auf das zur Verfügung gestellte Endprodukt des Plasmagenerators, und nicht auf ein Zwischenprodukt. Der maximale Ozon-Gehalt des Stickstoffmonoxid-haltigen Plasmas ist generell geringer als 5 ppm und in der Regel niedriger als 1 ppm, bevorzugt niedriger als 0,5 ppm und insbesondere geringer als 0,1 ppm. Andere Spezies als Ozon, beispielsweise Stickstoffdioxid, können im Stickstoffmonoxid-haltigen Plasma hingegen vorhanden sein. Umgekehrt ist das im zweiten Betriebsmodus erzeugte Ozon-haltige Plasma ein Plasma, das einen Anteil an Ozon enthält, während es im Wesentlichen frei von Stickstoffmonoxid ist und Letzteres allenfalls in praktisch nicht mehr nachweisbaren Spuren enthält. Der maximale Stickstoffmonoxid-Gehalt des Ozon-haltigen Plasmas ist generell geringer als 5 ppm und in der Regel niedriger als 1 ppm, bevorzugt niedriger als 0,5 ppm und insbesondere geringer als 0,1 ppm. Der zweite Betriebsmodus ist zweckmäßig dahingehend optimiert, dass der Anteil an Ozon im erzeugten Plasma möglichst hoch ist. Abgesehen von Stickstoffmonoxid kann das Ozon-haltige Plasma weitere Plasmaspezies wie erneut insbesondere Stickstoffdioxid enthalten.

Die erfindungsgemäße Vorrichtung ermöglicht es also, durch Auswahl eines der Betriebsmodi und der diesen zugeordneten unterschiedlichen Anregungsenergien unterschiedlich zusammengesetzte Plasmen zu erzeugen, von denen eines reich an Stickstoffmonoxid und das andere reich an Ozon ist. Die Verwendung der beiden unterschiedlichen Betriebsmodi erlaubt eine Behandlung sowohl mit Ozon als auch mit Stickstoffmonoxid, was bei herkömmlichen Plasmavorrichtungen bisher nicht möglich war. Mithilfe der Erfindung kann nun eine Hautpartie und insbesondere eine Wunde unter Verwendung ein und desselben Gerätes mit Plasmen unterschiedlicher Zusammensetzung und sowohl mit Stickstoffmonoxid als auch Ozon behandelt werden. Ozon ist für seine keimreduzierende Wirkung bekannt. Durch Auswahl des zweiten Betriebsmodus, der zur Erzeugung des Ozon-haltigen Plasmas führt, kann also eine besonders effektive Entkeimung der behandelten Hautpartie erreicht werden. Im ersten Betriebsmodus, in dem ein Stickstoffmonoxid-haltiges Plasma erzeugt wird, liegt die Hauptwirkung dagegen in einer beschleunigten Wundheilung. Die Auswahl des Betriebsmodus kann also beispielsweise in Abhängigkeit davon erfolgen, in welchem Zustand sich die zu behandelnde Hautpartie befindet. Bei hoher Keimbelastung wird zweckmäßig der zweite Betriebsmodus ausgewählt, um die Keimbelastung am Behandlungsort zu reduzieren. Danach kann beispielsweise eine weitere Behandlung im ersten Betriebsmodus erfolgen.

Die erfindungsgemäße Vorrichtung kann grundsätzlich so ausgebildet sein, dass ihr Benutzer selbst und nach eigenem Ermessen über die Auswahl des Betriebsmodus und/oder eine bestimmte Abfolge verschiedener Betriebsmodi nacheinander entscheiden kann. Dabei ist es möglich, dass der Benutzer nicht nur über die Art der Betriebsmodi, sondern auch über deren Anzahl und Abfolge nacheinander frei entscheiden kann. Weiter kann die Vorrichtung auch so ausgebildet sein, dass der Benutzer die Zeitdauer jedes einzelnen Behandlungsschritts in einem Betriebsmodus sowie die Gesamtdauer des Behandlungsvorgangs insgesamt auswählen kann. Möglich ist es jedoch auch, maximale Zeitdauern sowohl für einen einzelnen Behandlungsschritt als auch für die gesamte Behandlungsdauer vorzugeben, um eine Überbehandlung und eventuelle Schäden zu vermeiden. Alternativ oder ergänzend ist es möglich, feste Behandlungszyklen vorzugeben, in denen sich einzelne Behandlungsschritte in unterschiedlichen Betriebsmodi mit fest vorgegebenen Zeitdauern abwechseln. Dabei können Behandlungsschritte im ersten und im zweiten Betriebsmodus im Wechsel erfolgen oder auch mehrere Behandlungsschritte im selben Betriebsmodus aufeinander folgen, bevor mindestens ein Behandlungsschritt im anderen Betriebsmodus durchgeführt wird. Außerdem können Pausen gleicher oder unterschiedlicher Länge zwischen aufeinander folgenden Behandlungsschritten eingeschoben werden. Die erfindungsgemäße Vorrichtung erlaubt hier die verschiedensten Variationen in der Behandlung. Die in der Vorrichtung optional vorgegebenen festen Behandlungszyklen sind zweckmäßig in einem Speicher hinterlegt, auf den die Steuereinheit der erfindungsgemäßen Vorrichtung zugreifen kann. Außerdem kann die erfindungsgemäße Vorrichtung es dem Benutzer ermöglichen, von ihm selbst definierte Behandlungszyklen in dem Speicher zur späteren Verwendung abzuspeichern. Zur Auswahl der unterschiedlichen Betriebsmodi und zum Starten der Behandlung durch den Benutzer ist zweckmäßig eine Benutzerschnittstelle vorhanden, über welche die vom Benutzer eingegebenen Befehle an die Steuereinheit zur weiteren Verarbeitung übermittelt werden. Die Benutzerschnittstelle kann jede aus dem Stand der Technik bekannte Ausgestaltung aufweisen und beispielsweise in einem oder mehreren Schaltern, Wahlknöpfen, einem Touchscreen usw. bestehen. Außerdem kann auch eine Steuerung über ein externes Gerät wie beispielsweise ein Smartphone, Tablet oder einen PC vorgesehen sein.

Die erfindungsgemäße Vorrichtung ist zur Behandlung der Haut und insbesondere von Wunden vorgesehen. Dies schließt sowohl die Behandlung offener als auch geschlossener Wunden, beispielsweise die Behandlung von Narbengewebe oder Brandwunden, als auch die Behandlung entzündlicher Prozesse ein. Besonders geeignet ist die erfindungsgemäße Vorrichtung zur Behandlung chronischer Wunden, zum Beispiel Dekubitus- oder Diabetes-Wunden. Die Behandlung von Haut umfasst außerdem die Behandlung von Hauterkrankungen wie Ekzemen, Akne usw. Außerdem kann die Vorrichtung ebenfalls zur Behandlung von im Körper liegendem Gewebe verwendet werden. Beispiele möglicher Anwendungen sind in der WO 2015/066278 A1 beschrieben. Nachfolgend wird die Erfindung weiter am Beispiel der Wundbehandlung beschrieben. Diese Beschreibung ist jedoch nur beispielhaft und soll die Behandlung der vorstehend genannten Beispiele mit umfassen.

Die grundsätzliche Ausbildung der erfindungsgemäßen Vorrichtung entspricht generell derjenigen entsprechender Plasmaerzeugungsvorrichtungen aus dem Stand der Technik. Beispielsweise kann auf die Plasmavorrichtungen verwiesen werden, die in den vorstehend genannten Patentveröffentlichungen beschrieben sind. Bei der erfindungsgemäßen Vorrichtung kann es sich um eine Vorrichtung mit nur einer getriebenen Elektrode handeln, wobei die zweite Elektrode von der zu behandelnden Haut gebildet werden kann. Das auf diese Weise erzeugte Plasma wird dabei direkt über der Hautoberfläche erzeugt, was es entweder erforderlich macht, den Plasmagenerator in seiner Form an die dreidimensionale Form der zu behandelnden Oberfläche (zum Beispiel den Fuß eines Patienten) anzupassen oder mit einem Plasmagenerator geringer Oberfläche den zu behandelnden Bereich möglichst gleichmäßig abzuscannen. Zwar haben diesen Methoden den Vorteil, dass das Plasma praktisch ohne Aktivitätsverlust auf die Wunde einwirken kann, jedoch erfordern sie einen hohen Aufwand. Außerdem kann die unmittelbare Einwirkung des gerade erzeugten Plasmas auf die Wunde zu Zellschäden führen.

Ebenfalls einsetzbar sind Vorrichtungen, wie sie beispielsweise in der WO 2015/088948 A1 und WO 2018/127255 A1 beschrieben sind. In den beschriebenen Beispielen sind die Elektroden zur Erzeugung des Plasmas in eine Bandage oder ein Pflaster integriert, die unmittelbar am Behandlungsort befestigt werden. Dies hat zwar erneut den Vorteil, dass das Plasma beim Einwirken auf die Wunde eine hohe Aktivität aufweist. Nachteilig ist jedoch, dass die Anordnung der Elektroden unmittelbar am Patienten die Gefahr einer Beschädigung erhöht und zudem bei Fehlfunktionen der Patient selbst Gefahren, zum Beispiel durch Stromschläge, ausgesetzt ist. Außerdem wirken offene und vor allem hochfrequente und gepulste Plasmen als Antennen für elektromagnetische Störungen, die im medizinischen Behandlungsumfeld Fehlfunktionen anderer Geräte verursachen können und damit ebenfalls eine Gefahrenquelle darstellen.

Daher sind erfindungsgemäß solche Vorrichtungen bevorzugt, bei denen das Plasma in einem abgeschlossenen Volumen und entfernt von der zu behandelnden Wunde erzeugt und mittels einer Leitung zum Behandlungsort geführt wird. Somit tritt aus dem Plasmagenerator bei entsprechender Abschirmung oder Ableitung elektrischer bzw. elektromagnetischer Erscheinungen nur das Plasma heraus. Diese Vorrichtungen weisen generell wenigstens zwei Elektroden auf, die durch ein Dielektrikum voneinander getrennt sind. Das Plasma wird üblicherweise in einer Plasmakammer erzeugt, welche - abgesehen von einer Einlassöffnung für die Zufuhr der Ausgangsgasmischung in die Plasmakammer und einer Auslassöffnung, von welcher die Leitung für die Zufuhr des erzeugten Plasmas zum Behandlungsort ausgeht - luftdicht abgeschlossen ist. Außerdem ist zweckmäßig eine Gasversorgung vorhanden, um die Plasmakammer mit dem Ausgangsgas zu befüllen. Im einfachsten und erfindungsgemäß bevorzugten Fall handelt es sich dabei um die Zufuhr von Außenluft. Alternativ kann der Plasmakammer über einen oder mehrere Gasbehälter eine Gasmischung zugeführt werden, die Sauerstoff und Stickstoff enthält. Zur Förderung des Gases kann, falls erforderlich, eine Gastransportvorrichtung vorgesehen sein, bei der es sich zum Beispiel um einen Ventilator oder eine Pumpe handelt. Bei der Leitung, mit welcher das erzeugte Plasma dem Behandlungsort zugeführt wird, handelt es sich vorzugsweise um einen flexiblen Kunststoffschlauch aus einem gegenüber dem erzeugten Plasma beständigen Material. Vorrichtungen dieser Art sind beispielsweise in der WO 2018/189174 A1 und der EP 2914070 A1 beschrieben, auf deren Inhalt hier hinsichtlich der grundsätzlichen Ausbildung der Vorrichtung ausdrücklich Bezug genommen wird.

Das in der Plasmakammer erzeugte und dem Behandlungsort zugeleitete Plasma kann unmittelbar auf die Wunde appliziert werden. Dies ist wegen der kurzen Einwirkungszeit jedoch nicht bevorzugt. Vorteilhafter ist die aus dem Stand der Technik bereits bekannte Vorgehensweise, den Behandlungsort mit einer luftundurchlässigen Folie abzudecken, die oberhalb der Wunde ein Reservoir zur Aufnahme des Plasmagases bereitstellt. Die Anordnung ähnelt grundsätzlich einem luftdichten Pflaster, in das Plasmagas eingeleitet werden kann. Bevorzugt sind zumindest die Ränder des Pflasters selbstklebend, sodass die Folie in einfacher Weise auf der Haut des Patienten befestigt werden kann. In an sich bekannter Weise kann das Pflaster zudem Abstandshalter, Einlagen aus saugfähigem Material zur Aufnahme von Wundsekret oder Ähnliches aufweisen. Für die Einleitung von Plasma ebenfalls besonders gut geeignet sind Vakuumverbände, wie sie aus dem Stand der Technik für die Behandlung chronischer Wunden bekannt sind. Vorteilhaft beim Einschluss des Plasmas in einem abgeschlossenen Volumen oberhalb der Wunde ist es, dass das Plasma auf diese Weise eine längere Zeit an der Wunde lokalisiert bleibt und auch gut in Hohlstrukturen, Poren oder Haarwurzelscheiden eindringen kann, die bei anderer Wirkstoffapplikation praktisch nicht erreichbar sind.

In einer alternativen Ausführungsform der Erfindung wird das entweder mittels des ersten oder des zweiten Betriebsmodus produzierte Plasmagas nicht direkt auf die Wunde appliziert, sondern in eine Flüssigkeit eingeleitet. Bei der Flüssigkeit handelt es sich bevorzugt um eine wässrige Flüssigkeit, beispielsweise gereinigtes Wasser, eine Salzlösung, vorzugsweise eine physiologische Kochsalzlösung, oder eine Pufferlösung, wobei der pH-Wert der Pufferlösung zweckmäßig dem pH-Wert der Haut angepasst ist. Beim Einleiten des Plasmagases in die Flüssigkeit wird zumindest ein Teil der durch die dielektrische Barriereentladung produzierten reaktiven Spezies in der Flüssigkeit adsorbiert und/oder absorbiert. Die so erhaltene Flüssigkeit wird nachfolgend als "aktivierte Flüssigkeit" bezeichnet. Sie besitzt, abhängig von der Art des produzierten Plasmagases, entkeimende und/oder wundheilungsfördernde Wirkungen, welche sie bei entsprechender Lagerung mehrere Stunden, in der Regel mindestens etwa einen Tag lang, beibehält. Die aktivierte Flüssigkeit wird in üblicher Weise, beispielsweise durch Aufsprühen aus einer Sprühflasche, Auftropfen oder Aufpinseln, entweder direkt auf die Wunde oder auf ein auf der Wunde aufliegendes saugfähiges Pad appliziert und entfaltet dort die vorstehend genannten Wirkungen. Falls gewünscht, kann die Wunde anschließend mit einem Pflaster, einem Verband oder ähnlichem abgedeckt werden. Gegenüber der unmittelbaren Anwendung des Plasmagases auf die Wunde hat deren Behandlung mit aktivierter Flüssigkeit den Vorteil, dass die Wunde nicht austrocknet, was sich auf die Wundheilung positiv auswirkt. Aufgrund der Oberflächenspannung ist dagegen die Eindringtiefe in die Hautöffnungen geringer.

Ausbildung und Anordnung der wenigstens einen Elektrode zur Erzeugung der dielektrischen Barriereentladung entsprechen ebenfalls grundsätzlich dem aus dem Stand der Technik Bekannten. Die Elektrode kann beispielsweise ganzflächig oder gitterförmig/gelocht/gewickelt, plan oder gebogen, rohr- oder stabförmig ausgebildet sein. Sie besteht aus einem hochtemperaturfesten, elektrisch leitfähigen Material wie beispielsweise Wolfram. Bei Verwendung mehrerer Elektroden können gleich ausgebildete oder unterschiedliche Elektroden verwendet werden. Üblich ist beispielsweise die Verwendung zweier gegenüberliegender planer Elektroden, zwischen denen wenigstens ein Dielektrikum angeordnet ist, oder einer stabförmigen Elektrode, die von einem Zylinder aus nichtleitenden Material als Dielektrikum umgeben ist, auf dessen Außenumfang eine gitterförmige Elektrode gewickelt ist. Die Dielektrika bestehen beispielsweise aus Glas oder Keramik. Bei Verwendung mehrerer Dielektrika können diese gleich oder verschieden sein.

Versorgungseinheit und Steuereinheit zur Steuerung der Versorgungseinheit entsprechen ebenfalls dem Stand der Technik. Die Versorgungseinheit erzeugt entweder Wechselspannung oder gepulste Gleichspannung, wobei die Verwendung gepulster Gleichspannung bevorzugt ist. Für eine Vorrichtung eines vorgegebenen Aufbaus lässt sich die Zusammensetzung des erzeugten Plasmagases über die zugeführte Energie steuern. Durch eine geeignete Festlegung der elektrischen Betriebsparameter für den ersten und zweiten Betriebsmodus kann daher erreicht werden, dass im ersten Fall ein Stickstoffmonoxid-haltiges Plasma und im zweiten Fall ein Ozon-haltiges Plasma erzeugt wird. In welcher Weise die elektrischen Betriebsparameter gewählt werden müssen, hängt vom Aufbau des jeweiligen Plasmagenerators ab. In allen Fällen ist es jedoch so, dass die Anregungsenergie zur Erzeugung eines Stickstoffmonoxid-haltigen Plasmas größer ist als diejenige, die zur Erzeugung eines Ozon-haltigen Plasmas erforderlich ist.

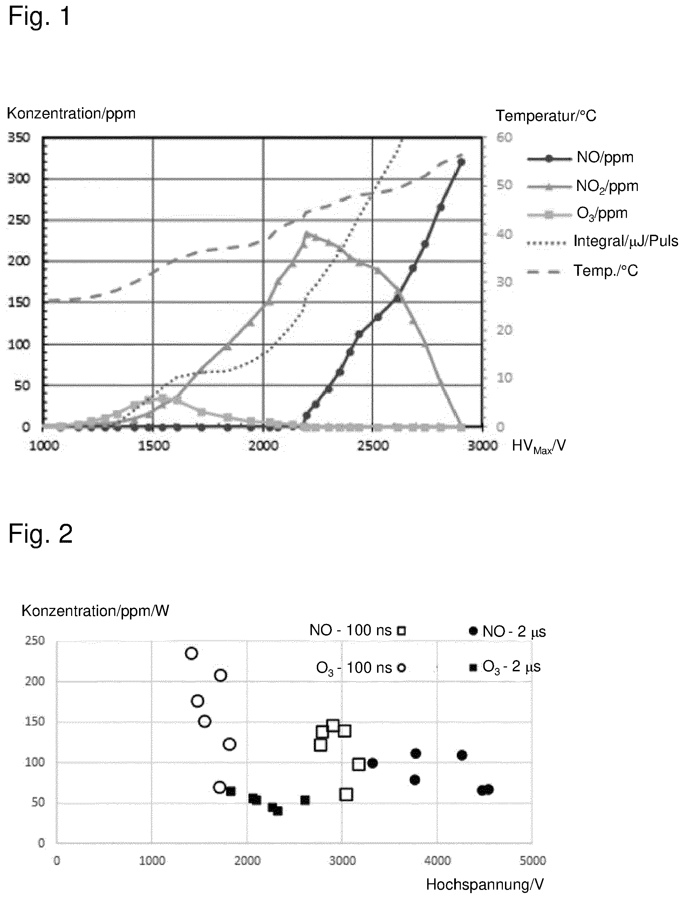

Figur 1 zeigt die grundsätzliche Abhängigkeit der Plasmazusammensetzung von der zugeführten Anregungsenergie für die Spezies Stickstoffmonoxid, Stickstoffdioxid und Ozon. Für eine vorgegebene Geometrie des Plasmagenerators und eine vorgegebene Anregungsfrequenz hängt die Zusammensetzung des Plasmas nur noch von der angelegten Barrierespannung (HV_{Max}) ab. Diese muss so weit angehoben werden, dass die typischen Mikroentladungen der Barriereentladung vom Charakter einer Glimmentladung zu einer reinen Streamer-Entladung getrieben werden können. Dazu sind hohe Feldstärken mit hohen Streamerströmen, also hohe Leistungen, notwendig. Die einzubringende Energie steht unmittelbar mit den unterschiedlichen Aktivierungsenergien zum Trennen von Stickstoff- und Sauerstoffbindungen im Ausgangsgas in Verbindung. Wie sich der Figur entnehmen lässt, wird bei einer niedrigen Barrierespannung (im gezeigten Beispiel unterhalb von 2000 V) Ozon gebildet. Daneben bildet sich ab ca. 1400 V auch Stickstoffdioxid. Ab einer Barrierespannung von etwa 2200 V steigt die Stickstoffdioxid-Produktion nicht weiter an, sondern fällt mit zunehmender Spannung ab, bis sie bei einer Spannung von ca. 2900 V zum Erliegen kommt. Stickstoffmonoxid wird überhaupt erst ab einer Spannung von ca. 2200 V gebildet. Ab einer Spannung von ca. 2700 V ist Stickstoffmonoxid die überwiegend gebildete Spezies im Plasma. Figur 1 zeigt also, dass keine Plasmen gebildet werden, in denen Stickstoffmonoxid und Ozon gemeinsam enthalten sind und dass sich durch geeignete Auswahl der Barrierespannung Plasmen erzeugen lassen, die entweder Ozon oder Stickstoffmonoxid enthalten.

Die Barrierespannung, die angelegt werden muss, um entweder ein Stickstoffmonoxid-reiches oder ein Ozon-reiches Plasma zu erzeugen, hängt, wie erwähnt, von der Ausgestaltung des Plasmagenerators sowie vom verwendeten Ausgangsgas ab. Im Rahmen der Erfindung wurde daher versucht, eine Definition für die zuzuführende Energie zu finden, welche weitgehend unabhängig von diesen Parametern ist. Entsprechend wird die erforderliche Anregungsenergie für den ersten und den zweiten Betriebsmodus bevorzugt auf der Basis der Plasmaleistung, bezogen auf die Entladungsfläche, bestimmt. Unter der Plasmaleistung ist das Produkt der gemessenen Entladungsspannung (der an der Barriere anliegenden Spannung) und des durch die Entladungsspannung ausgelösten und gemessenen Entladungsstroms zu verstehen. Die Plasmaleistung muss von der Blindleistung, die durch die Umleitung der Barrierekapazitäten entsteht, unterschieden werden. Diese Unterscheidung ist gängige Praxis und wird zusammen mit den entsprechenden Messmethoden ausführlich in der Literatur beschrieben (A.V. Pipa et al. "The Equivalent Circuit Approach for the Electrical Diagnostics of Dielectric Barrier Discharges: The Classical Theory and Recent Developments", Atoms (2019) 7, 14 (doi:10.3390/atoms7010014); F. Peeters et al. "Electrical Diagnostics of Dielectric Barrier Discharges", DOI:http://dx.doi.org/10.5772/intechopen.80433). Die Entladungsfläche ist diejenige Fläche, über die eine geschlossene Entladung auftritt. Üblicherweise findet die Entladung bei Erreichen der Durchbruchsspannung in Form von zahlreichen über die Elektrodenfläche stochastisch verteilten Mikroentladungen statt. Die Entladungsfläche ist also die Gesamtfläche aller von den Mikroentladungen eingenommenen Teilflächen. Da die Entladung mit einer Lichtemission verbunden ist, wird die Entladungsfläche unmittelbar erkennbar. Nicht leuchtende Flächen zählen somit nicht zur Entladungsfläche. Die Entladungsfläche kann in einfacher Weise beispielsweise durch bildgebende Verfahren ermittelt werden. Zweckmäßig werden dabei für den verwendeten Plasmagenerator mehrere Entladungsvorgänge beobachtet und mehrere Bilder angefertigt und ausgewertet, um so einen Mittelwert über die Ergebnisse bilden zu können und die Genauigkeit zu erhöhen. Aufgrund der im Rahmen der Erfindung durchgeführten Untersuchungen an verschiedenen Plasmageneratoren haben sich folgende bevorzugte Plasmaleistungen, bezogen auf die Entladungsfläche, für den ersten und den zweiten Betriebsmodus ergeben: Für den ersten Betriebsmodus, der zu einem Stickstoffmonoxid-haltigen Plasma führt, liegt die erste Anregungsenergie bei über 0,25 W/cm². Besonders bevorzugt beträgt sie mehr als 0,4 W/cm². Für den zweiten Betriebsmodus und die Herstellung eines Ozon-haltigen Plasmas ist die zweite Anregungsenergie geringer und liegt zweckmäßig im Bereich von 0,01 bis 0,25 W/cm², bevorzugt bei 0,01 bis 0,1 W/cm². Die angegebenen Werte beziehen sich auf das erfindungsgemäß bevorzugte Ausgangsgas, Luft. Geeignete Anregungsenergien für andere Sauerstoff und Stickstoff enthaltende Ausgangsgase können vom Fachmann jedoch durch einfache Versuche ermittelt werden.

Die als Plasmaleistung in Bezug auf die Entladungsfläche bemessene Anregungsenergie ermöglicht es, die Anregungsenergien für den ersten und den zweiten Betriebsmodus für praktisch jede Art von Plasmagenerator durch einfache Bestimmung der Entladungsfläche vorab zu ermitteln und die elektrischen Betriebsparameter so einzustellen, dass der Plasmagenerator in den erfindungsgemäßen zwei Betriebsmodi unter Bildung eines Stickstoffmonoxid-haltigen Plasmas einerseits und eines Ozon-haltigen Plasmas andererseits betrieben werden kann. Die erfindungsgemäß definierten Bereiche für die Plasmaleistung in Bezug auf die Entladungsfläche liefern dabei grundsätzlich einen ersten Anhaltspunkt zur Einstellung der elektrischen Betriebsparameter eines Plasmagenerators. Gegebenenfalls kann jedoch für einen optimierten Betrieb noch eine Anpassung und Feinjustierung der Einstellungen erforderlich sein.

Die Optimierung der elektrischen Betriebsparameter eines Plasmagenerators kann insbesondere im Hinblick auf eine Steigerung des Anteils der gewünschten reaktiven Spezies Stickstoffmonoxid einerseits und Ozon andererseits, die Reduzierung nicht erwünschter Spezies im Plasma, die Temperatur des Plasmas und/oder den Energieverbrauch erfolgen. Generell ist es für beide Betriebsmodi bevorzugt, die Anregungsenergie in Form einer gepulsten Gleichspannung zuzuführen. Bei der Optimierung des Ozon-Gehalts im zweiten Betriebsmodus hat sich gezeigt, dass die Pulsfrequenz einen deutlichen Einfluss auf die Ozonkonzentration besitzt. Untersuchungen haben ergeben, dass tendenziell kürzere Pulse zu einem größeren Anteil an Ozon im Plasma führen. Dies lässt sich vermutlich darauf zurückführen, dass eine höhere Pulsfrequenz die Entladung von einer filamentierten Entladung hin zu einer homogeneren Entladung verschiebt. Erfindungsgemäß ist es daher bevorzugt, wenn für die zweite Anregungsenergie die Pulslänge kürzer als 1 *µ*s ist. Als besonders vorteilhaft hat es sich herausgestellt, die zweite Anregungsenergie in Form von Pulsgruppen zuzuführen. Die in einer Pulsgruppe zusammengefassten Pulse sind dabei jeweils kürzer als 1 *µ*s. In der Pause zwischen einzelnen Pulsgruppen wird der Barriere keine Energie zugeführt. Als übergeordnetes Prinzip bei der Zufuhr der gepulsten Energie sollte jedoch weiterhin die auf die Entladungsfläche bezogene Plasmaleistung beachtet werden, die für die Erzeugung eines Ozon-haltigen Plasmas, wie erwähnt, im Bereich von 0,01 bis 0,25 W/cm² liegen sollte.

Der Zusammenhang zwischen der Pulsdauer und der Bildung von Stickstoffmonoxid und Ozon für verschiedene Barrierespannungen ist in Figur 2 dargestellt. Die offenen Kreise stehen dabei für Ozon, das mit Pulsen von 100 ns Länge erzeugt wurde, die schwarzen Quadrate für Ozon, für dessen Erzeugung Pulse von 2 *µ*s Länge verwendet wurden. Während in letzterem Fall Ozon in einer Menge von ungefähr 50 ppm/Watt erzeugt wurde, liegt die Ozonkonzentration bei Verwendung von Pulsen mit 100 ns Länge deutlich darüber, teilweise beim Vierfachen. Aus der Figur ist ebenfalls ersichtlich, dass die Pulsfrequenz bei der Erzeugung von Stickstoffmonoxid eine deutlich geringere Rolle spielt. Die durch die offenen Quadrate gekennzeichnete Konzentration von Stickstoffmonoxid, dass mit Pulsen von 100 ns Länge generiert wurde, überschreitet nur zum Teil, und dann auch nur wenig, die Konzentration von Stickstoffmonoxid, dass mit Pulsen von 2 *µ*s Länge erzeugt wurde (schwarze Punkte). Dies dürfte darauf zurückzuführen sein, dass die Bildung von Stickstoffmonoxid endotherm verläuft. Es ist bekannt, dass für die Plasmaerzeugung von Stickstoffmonoxid grundsätzlich ein heißer Kanal, meist als Lichtbogen zwischen zwei Elektroden, existieren muss. Die aufgrund dessen unerwünscht hohen Temperaturen des Plasmagases können durch die Vermischung mit kaltem Prozessgas und/oder die Begrenzung der Entladungszeit und des Entladungsstroms vermindert werden. Dies führt dann allerdings zu einer verringerten Effizienz der NO-Erzeugung und einer geringeren absoluten Stickstoffmonoxidkonzentration. Insgesamt wird die Bildung von Stickstoffmonoxid also vor allem vom Energieeintrag dominiert. Dies ergibt sich auch aus der gepunkteten Linie in Figur 1, in der die Pulsenergie in *µ*J/Puls angegeben ist. Die gepunktete Linie steigt parallel zu der Bildung von Stickstoffmonoxid an. In diesem Bereich ist der Aufwand für die elektromagnetische Verträglichkeit (hochfrequenter Anteil durch kurze Pulse und hohe Leistung) erheblich und kann zum Beispiel durch geringere Frequenzen mit bipolarer homogener Anregung (Sinuswelle) ohne besondere Effizienzverluste verringert werden.

Die Erfindung betrifft weiterhin ein Verfahren zur Behandlung der Haut und insbesondere einer Wunde unter Verwendung der vorstehend beschriebenen Vorrichtung. Das Verfahren ist gekennzeichnet durch die folgenden Schritte:
- Auswählen eines der Betriebsmodi aus dem ersten und dem zweiten Betriebsmodus,
- Steuern der Versorgungseinheit durch die Steuereinheit entsprechend dem ausgewählten Betriebsmodus, sodass der wenigstens einen Elektrode die erste Anregungsenergie oder die zweite Anregungsenergie zugeführt wird.
Abhängig von der Art des verwendeten Plasmagenerators kann das Verfahren optional wenigstens einen der weiteren Schritte umfassen:
- Einleiten von Gas in eine Plasmakammer mittels einer Gastransportvorrichtung,
- Zuführen eines Plasmagases aus der Plasmakammer über eine Leitung zum Behandlungsort oder
- Zuführen eines Plasmagases aus der Plasmakammer in einen mit einer Flüssigkeit, insbesondere einer wässrigen Flüssigkeit, befüllten Behälter.

Wie bereits in Zusammenhang mit der Vorrichtung diskutiert, kann die Auswahl zwischen erstem und zweitem Betriebsmodus entweder vom Bediener der Vorrichtung vorgenommen werden, wozu zweckmäßig die bereits zuvor beschriebene Benutzerschnittstelle verwendet werden kann. Alternativ oder zusätzlich ist es möglich, dass die Auswahl zwischen den Betriebsmodi automatisch erfolgt - sei es nach einem bereits bei der Herstellung der Vorrichtung programmierten Auswahlmodus oder einem Auswahlmodus, den der Benutzer vorgegeben und in der Speichereinheit der Vorrichtung abgespeichert hat. Entsprechend der Auswahl des Betriebsmodus steuert die Steuereinheit dann die Versorgungseinheit so, dass diese bei Auswahl des ersten Betriebsmodus der wenigstens einen Elektrode die erste Anregungsenergie zugeführt und bei Auswahl des zweiten Betriebsmodus die zweite Anregungsenergie. Die Dauer des Betriebs im ausgewählten Betriebsmodus kann entweder vom Benutzer vorgegeben werden oder nach in der Speichereinheit der Vorrichtung abgespeicherten Vorgaben erfolgen. Die besonderen Vorteile der erfindungsgemäßen Vorrichtung und des Verfahrens kommen dann zur Geltung, wenn die Behandlung in einer Abfolge von Behandlungsschritten mit unterschiedlichen Betriebsmodi erfolgt. Die vielfältigen Variationsmöglichkeiten wurden ebenfalls bereits in Zusammenhang mit der Vorrichtung beschrieben. Sie erlauben eine längerfristige Wundbehandlung, bei der die Wunde sowohl entkeimt als auch ihre Heilung beschleunigt werden kann. Durch die Verwendung von dielektrischen Barriereentladung zur Erzeugung der unterschiedlichen Plasmen und die dem Verfahren inhärente Strombegrenzung werden sowohl im ersten als auch im zweiten Betriebsmodus jeweils Plasmen mit reproduzierbarer Zusammensetzung erzeugt, was wiederum eine genaue Dosierung und Kontrolle der Behandlungsschritte ermöglicht.

Zur Erzeugung der Zielspezies Stickstoffmonoxid und Ozon wird als Ausgangsgas eine Sauerstoff und Stickstoff enthaltende Gasmischung verwendet. Besonders bevorzugt wird Luft eingesetzt. Gute Ergebnisse werden erzielt, wenn die Gasmischung wenigstens eine der folgenden Eigenschaften aufweist: eine Temperatur von 15 bis 60 °C, eine relative Feuchte von 20 bis 90 %, einen Schwebstoffgehalt von 0,1 bis 20 mg/m³ und einen Durchfluss von 0,5 bis 10 slm (Standard-Liter pro Minute). Höhere Temperaturen sind vor allen Dingen im ersten Betriebsmodus zur Herstellung von Stickstoffmonoxid sinnvoll, da dessen Bildung endotherm abläuft. Die Temperatur der Gasmischung ist in Figur 1 mit der gestrichelten Linie verdeutlicht. Wie erkennbar, steigt die Temperatur in Richtung auf höhere Konzentrationen von Stickstoffmonoxid an. Eine höhere relative Feuchte der Gasmischung ist ebenfalls geeignet, den Anteil reaktiver Spezies im Plasma in Richtung auf einen höheren Stickstoffmonoxid-Anteil hin zu verschieben. Der Grund dafür ist, dass gebildetes Ozon und andere reaktive Sauerstoffspezies mit dem in der Gasmischung vorhandenen Wasser unter Bildung von Sauerstoff und Wasserstoffperoxid reagieren, wodurch die Konzentration von Ozon im Plasma sinkt. Da Ozon ansonsten mit Stickstoffmonoxid reagieren würde, steigt die Stickstoffmonoxidkonzentration entsprechend schneller an. Ein hoher Schwebstoffgehalt führt zu einer schnelleren Deaktivierung der reaktiven Spezies, weshalb es grundsätzlich bevorzugt ist, dass der Schwebstoffgehalt möglichst niedrig ist. Der Durchfluss wird zweckmäßig so bemessen, dass das Ausgangsgas genügend lange im Bereich der wenigstens einen Elektrode verbleibt, um ausreichend Plasma zu bilden. Andererseits sollte bei Verwendung einer Leitung, um Plasma aus der Plasmakammer zum Behandlungsort zu transportieren, die Transportdauer möglichst kurz sein, da die Konzentration an reaktiven Spezies im Plasma relativ schnell absinkt. Wie genau der Durchfluss eingestellt wird, hängt überwiegend von der Art und Geometrie der verwendeten Vorrichtung sowie der Konzentration der gebildeten reaktiven Spezies ab. Er kann jedoch vom Fachmann auf einfache Weise so eingestellt werden, dass am Behandlungsort die gewünschte Konzentration der reaktiven Spezies vorhanden ist. Dies kann beispielsweise durch geeignete Einstellung der Gastransportvorrichtung erfolgen, konkret zum Beispiel durch Einstellung der Förderleistung eines Ventilators oder einer Pumpe.

Wie ebenfalls bereits in Zusammenhang mit der Vorrichtung beschrieben, verläuft zweckmäßig auch das erfindungsgemäße Verfahren derart, dass die Versorgungseinheit bei Auswahl des ersten Betriebsmodus die erste Anregungsenergie mit mehr als 0,25 W/cm², bevorzugt mehr als 0,4 W/cm², und bei Auswahl des zweiten Betriebsmodus die zweite Anregungsenergie im Bereich von 0,01 bis 0,25 W/cm², bevorzugt 0,01 bis 0,1 W/cm², jeweils angegeben als Plasmaleistung in Bezug auf die Entladungsfläche, zuführt. Vorzugsweise führt die Versorgungseinheit die Anregungsenergie als gepulste Spannung und insbesondere als gepulste Gleichspannung zu. Für den Betrieb im zweiten Betriebsmodus hat es sich als besonders vorteilhaft erwiesen, wenn die zweite Anregungsenergie in Form von Pulsgruppen zuführt wird, in denen mehrere Pulse zusammengefasst sind. Jeder der Einzelpulse ist dabei zweckmäßig kürzer als 1 *µ*s und beträgt beispielsweise zwischen 20 und 500 ns, bevorzugt 50 bis 250 ns und insbesondere 100 ns.

Die Barrierespannung, die an die wenigstens eine Elektrode angelegt werden muss, um die erforderliche Durchschlagsspannung zur Erzeugung der Mikroentladungen zu erreichen, ist stark vom Aufbau des verwendeten Plasmagenerators abhängig. Geeignete Barrierespannungen liegen üblicherweise im kV-Bereich. Lediglich um ein Beispiel für eine erfindungsgemäß bevorzugte Vorrichtung zu geben, sollen hier die verwendeten Barrierespannungen genannt werden, die für den ersten Betriebsmodus zwischen 2000 und 4000 V, insbesondere bei 2500 bis 3500 V, und/oder für den zweiten Betriebsmodus zwischen 900 und 2000 V, insbesondere bei 1000 bis 1700 V, liegen. Wie bereits gesagt, können die Barrierespannungen für anders aufgebaute Vorrichtungen jedoch deutlich von diesen Werten abweichen.

Zusätzlich zur Beeinflussung der Zusammensetzung des Plasmas über die Energiezufuhr können auch weitere geeignete Maßnahmen ergriffen werden, um die Plasmazusammensetzung zu ändern. Auf den Einfluss der Luftfeuchtigkeit auf die Plasmazusammensetzung wurde bereits hingewiesen. Weiterhin ist es möglich, Filter zu verwenden, die bestimmte reaktive Spezies aus dem gebildeten Plasma entfernen. Auf die Möglichkeit der Verwendung eines Stickstoffdioxid-Filters wurde bereits hingewiesen (vergleiche WO 2013/052548 A2). Ebenfalls denkbar ist die katalytische Zersetzung nicht erwünschter Spezies, um diese aus dem Plasma zu entfernen. UV-Filter können verwendet werden, was jedoch nur erforderlich ist, wenn die Entladungsprozesse und damit die Bildung von UV-Strahlung in unmittelbarer Nähe zum Behandlungsort erfolgen.

Die Erfindung soll nachfolgend am Beispiel von Zeichnungen näher erläutert werden. Die Zeichnungen sind lediglich schematisch dienen der Beschreibung einiger bevorzugter Ausführungsbeispiele der Erfindung, ohne dass die Erfindung auf diese Beispiele beschränkt wäre. Gleiche Bezugszeichen bezeichnen gleiche Teile, wobei nicht alle Teile mit einem Bezugszeichen versehen sein müssen. In den Figuren zeigen:
- Fig. 1: ein Diagramm, welches die Konzentration einiger reaktiver Spezies im Plasmagas in Abhängigkeit von der angelegten Barrierespannung verdeutlicht,
- Fig. 2: ein Diagramm, in welchem die Konzentration von Stickstoffmonoxid und Ozon im Plasma in Abhängigkeit von der angelegten Barrierespannung und der Pulsdauer verdeutlicht,
- Fig. 3: den Grundaufbau eines ersten Ausführungsbeispiels einer erfindungsgemäßen Vorrichtung,
- Fig. 4: den Grundaufbau eines zweiten Ausführungsbeispiels einer erfindungsgemäßen Vorrichtung,
- Fig. 5: ein erstes Ausführungsbeispiel einer Elektrodenanordnung zur Verwendung in einer erfindungsgemäßen Vorrichtung,
- Fig. 6: ein zweites Ausführungsbeispiel einer Elektrodenanordnung zur Verwendung in einer erfindungsgemäßen Vorrichtung,
- Fig. 7: ein drittes Ausführungsbeispiel einer Elektrodenanordnung zur Verwendung in einer erfindungsgemäßen Vorrichtung und
- Fig. 8: ein viertes Ausführungsbeispiel einer Elektrodenanordnung zur Verwendung in einer erfindungsgemäßen Vorrichtung.

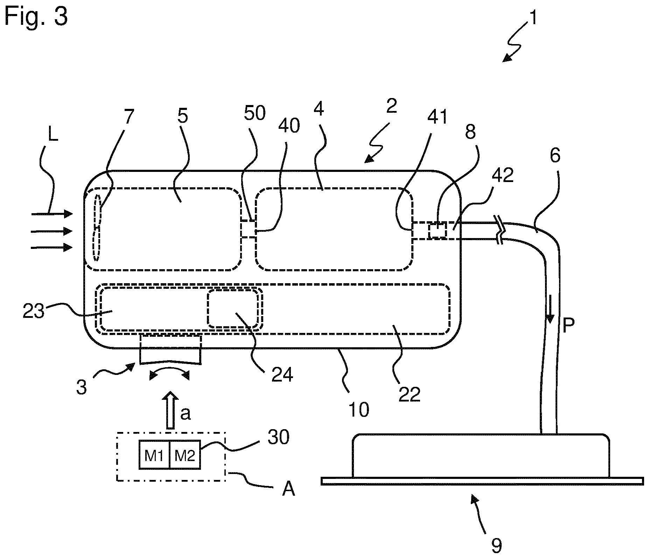

Figur 3 verdeutlicht stark schematisiert einen möglichen Grundaufbau einer erfindungsgemäßen Vorrichtung 1. Die Vorrichtung umfasst ein Gehäuse 10, das hier in Seitenansicht gezeigt ist. Die darin angeordneten Komponenten sind gestrichelt dargestellt. Im linken stirnseitigen Ende des Gehäuses 10 befindet sich ein in der Darstellung nicht sichtbarer Einlass, beispielsweise in Form von mehreren Lüftungsschlitzen, für das Ausgangsgas, hier Luft. Die Luft L gelangt nach Durchtritt durch den Einlass zu einer Gasversorgung 5, die in einer Gasvorratskammer besteht, in welcher eine Gastransportvorrichtung 7 in Form eines Ventilators angeordnet ist. Der Ventilator fördert die Luft in die Gasvorratskammer und aus dieser weiter in einen Plasmagenerator 2, der mit der Gasversorgung 5 über eine Leitung 50 in Verbindung steht. Der Plasmagenerator 2 umfasst eine Plasmakammer 4, die abgesehen von einem mit der Leitung 50 in Verbindung stehenden Einlass 40 und einem Auslass 41 auf der gegenüberliegenden Seite luftdicht abgeschlossen ist. In der Plasmakammer 4 wird Plasmagas durch eine dielektrische Barriereentladung erzeugt. Dazu sind in der Plasmakammer 4 hier der Übersichtlichkeit halber nicht dargestellte Elektroden angeordnet, die durch wenigstens ein Dielektrikum voneinander getrennt sind. Zur Erzeugung der dielektrischen Barriereentladung wird an die Elektroden mittels einer elektrischen Versorgungseinheit 22 eine elektrische Entladungsspannung angelegt. Die Steuerung der Versorgungseinheit 22 erfolgt mittels einer Steuereinheit 23, die wiederum über eine Benutzerschnittstelle 3 von einem Benutzer der Vorrichtung bedient werden kann. Die Benutzerschnittstelle 3 umfasst im gezeigten Beispiel einen Wippentaster 30. Er ist in der Detailansicht A in Draufsicht in Richtung des Pfeils a gezeigt. Wird der Wippentaster 30 vom Benutzer durch Druck auf die linke, mit M1 gekennzeichnete Wippenseite betätigt, steuert die Steuereinheit 23 die Versorgungseinheit gemäß einem ersten Betriebsmodus M1, der in einem Speicher 24 abgespeichert ist. Die Versorgungseinheit 22 führt den Elektroden eine erste Anregungsenergie zu, die ausreicht, um ein Stickstoffmonoxid-haltiges Plasma zu erzeugen. Erfindungsgemäß liegt diese erste Anregungsenergie bevorzugt in einem Bereich von mehr als 0,25 W/cm², angegeben als Plasmaleistung, bezogen auf die Entladungsfläche. Wird der Wippentaster 30 vom Benutzer durch Druck auf die rechte, mit M2 gekennzeichnete Wippenseite betätigt, steuert die Steuereinheit 23 dagegen die Versorgungseinheit 22 gemäß einem ebenfalls im Speicher 24 gespeicherten zweiten Betriebsmodus M2. In diesem wird den Elektroden eine zweite Anregungsenergie zugeführt, die geringer ist als die erste Anregungsenergie und insbesondere in einem Bereich von 0,01 bis 0,25 W/cm² liegt, sodass ein Ozon-haltiges Plasma gebildet wird. Das gebildete Plasmagas P wird anschließend aus der Plasmakammer 4 heraus durch eine Auslassleitung 42 transportiert. In der Auslassleitung 42 ist im gezeigten Beispiel ein Filter 8 angeordnet, der Stickstoffdioxid (NO₂) aus dem Plasmagas herausfiltert. Über eine an die Auslassleitung 42 angeschlossene Leitung 6 in Form eines Schlauches wird das Plasmagas P dann zu dem Behandlungsort (der zu behandelnden Wunde) transportiert und auf die Wunde appliziert. Dies geschieht im gezeigten Beispiel derart, dass das von der Plasmakammer entfernt gelegene Ende des Schlauches 6 in einen luftdichten Verband 9 einmündet, der auf der Hautoberfläche eines Patienten über der Wunde luftdicht befestigt ist. Das Plasma wird über einen längeren Zeitraum, beispielsweise von mehreren Stunden bis hin zu mehreren Tagen oder Wochen, kontinuierlich oder intermittierend erzeugt und in den Verband 9 eingeleitet. Dabei kann mehrfach zwischen den verschiedenen Betriebsmodi hin und her gewechselt werden, sei es durch Betätigung des Wippentasters 30 durch den Benutzer oder automatisch durch die Steuereinheit 23 vorgegeben, sodass sich die Zusammensetzung des Plasmas im Verlauf der Behandlung ändert. Ozon-haltiges Plasma dient dabei in erster Linie der Desinfektion der behandelten Wunde, Stickstoffmonoxid-haltiges Plasma bewirkt hauptsächlich einen beschleunigten Heilungsprozess.

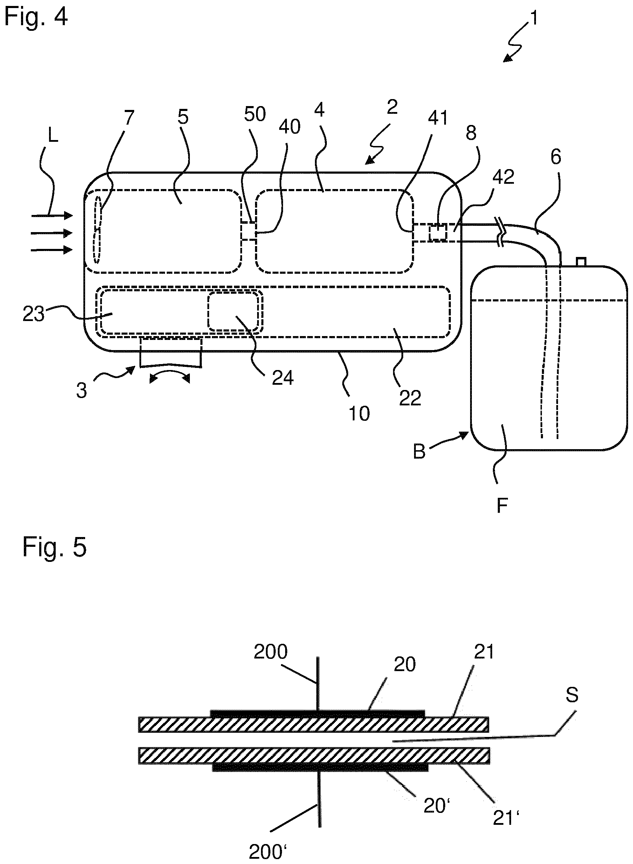

Figur 4 zeigt ein zweites Ausführungsbeispiel einer erfindungsgemäßen Vorrichtung 1. Der Aufbau der Vorrichtung entspricht demjenigen der in Figur 3 dargestellten Vorrichtung. Der Unterschied zwischen den Vorrichtungen besteht in der weiteren Verwendung des im Plasmagenerator 2 erzeugten Plasmagases. In der Vorrichtung gemäß Figur 4 wird das Plasmagas P nach Verlassen des Plasmagenerators 2 ebenfalls über die Auslassleitung 42 in eine Leitung 6 in Form eines Schlauches eingeleitet, dann jedoch nicht direkt einer Wunde zugeführt, sondern in einen mit Flüssigkeit F befüllten Behälter B eingeleitet. Bei der Flüssigkeit F handelt es sich vorzugsweise um eine wässrige Flüssigkeit wie eine physiologische Kochsalzlösung oder eine Pufferlösung. Die im Plasmagas P enthaltenen, durch die dielektrische Barriereentladung gebildeten aktiven Spezies werden in der wässrigen Lösung zumindest teilweise adsorbiert und/oder absorbiert und führen zur Bildung einer aktivierten Flüssigkeit. Deren Zusammensetzung ändert sich in Abhängigkeit davon, ob das Plasmagas P im ersten Betriebsmodus M1 oder im zweiten Betriebsmodus M2 hergestellt wurde. Die aktivierte Flüssigkeit kann entweder direkt aus dem Behälter B auf die zu behandelnde Wunde appliziert werden, oder sie wird zunächst in einen geeigneten Applikator, beispielsweise eine Sprühflasche, umgefüllt.

Figuren 5 bis 8 beschreiben verschiedene Elektrodenanordnungen, die in den Plasmageneratoren der erfindungsgemäßen Vorrichtungen eingesetzt werden können. In der Anordnung gemäß Figur 5 sind zwei plane und öffnungsfreie Metallplatten als Elektroden 20, 20' gegenüberliegend angeordnet und über elektrische Leitungen 200, 200' mit der hier nicht dargestellten Versorgungseinheit 22 verbunden. Zwischen den Elektroden 20, 20' sind zwei ebenfalls flache und öffnungsfreie Dielektrika 21, 21' aus einem nicht leitenden Material, beispielsweise Glas oder Keramik, angeordnet. Beim Erreichen der Durchbruchsspannung findet im Spalt S zwischen den Dielektrika 21, 21' eine dielektrische Barriereentladung statt, die zur Bildung des Plasmagases und der darin enthaltenen reaktiven Spezies führt. Je nachdem, ob den Elektroden 20, 20' die erste oder die zweite Anregungsenergie zugeführt wird, bildet sich dabei entweder ein Stickstoffmonoxid-haltiges Plasma oder ein Ozon-haltiges Plasma.

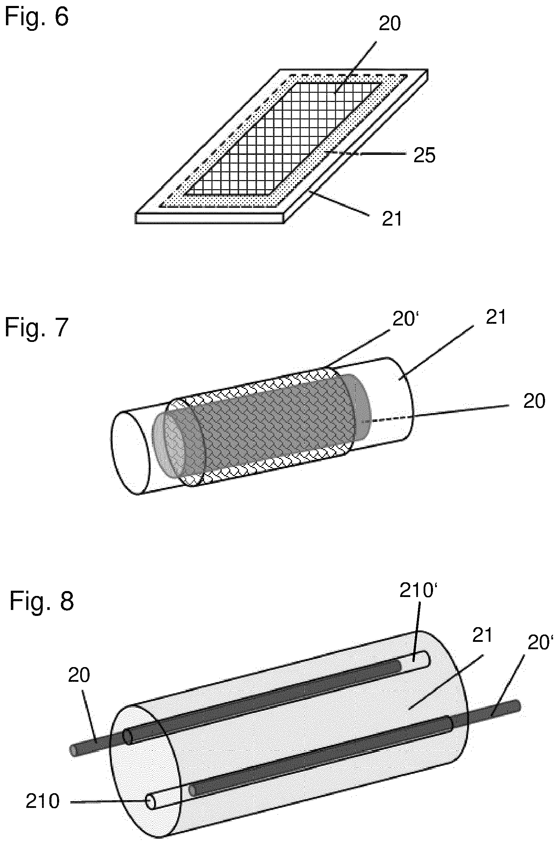

Figur 6 zeigt eine alternative Elektrodenanordnung mit einer Gitterelektrode 20, die auf einem geschlossenen Rückkontakt 25 angeordnet ist, der wiederum auf einer dielektrischen Platte als Dielektrikum 21 aufliegt. In diesem Fall erfolgt die Entladung als Oberflächen-Mikroentladung (SMD = surface microdischarge). Die Gitterelektrode 20 kann dabei unterschiedlichste Formen aufweisen, sowohl was ihre Außenform als auch die Ausgestaltung des Gitters anbetrifft. Die Außenform ist grundsätzlich beliebig und kann beispielsweise rechteckig, quadratisch, mehreckig oder auch gerundet sein. Als Gitter kommen Quadrat-, Rechteck- oder Rautengitter sowie Loch- und Wabengitter, aber auch Kamm- und maeanderartige Strukturen infrage. Das Plasma entsteht an den Strukturkanten zur dielektrischen Platte 21.

Die in Figur 7 gezeigte Elektrodenanordnung weist eine stab- oder rohrförmige innere Elektrode 20 auf, die von einem hohlzylindrischen Dielektrikum 21 umgeben ist. Auf den Außenumfang des Dielektrikums 21 ist eine Außenelektrode 20' in Form eines Strumpfes aus Metallgeflecht oder Metallgestrick gezogen. Alternativ könnte die Außenelektrode auch von einer Lochfolie, einer Drahtwicklung oder ähnlichem gebildet sein. Die Innenelektrode 20 kann alternativ auch aus einer Metallbeschichtung auf der inneren Oberfläche des hohlzylindrischen Dielektrikums 21 oder einer dort angeordneten Metallfolie bestehen. Auch Formen der Elektroden 20, 20' sind denkbar, die nur Teilbereiche des Umfangs des Dielektrikums 21 bedecken, solange sich die Elektroden auf beiden Seiten des Dielektrikums überlappen. Die Entladung bildet sich erneut an den Strukturkanten der Elektroden zum Dielektrikum 21.

In der Elektrodenanordnung, die in Figur 8 gezeigt ist, wird das Dielektrikum 21 von einem nichtleitenden Zylinder, beispielsweise aus Glas, gebildet, in dessen Längsrichtung sich zwei Längsbohrungen 210, 210' erstrecken, in die jeweils eine stabförmige Elektrode 20, 20' eingeschoben ist. Ein Ende der Elektroden steht jeweils über eine Stirnseite des Zylinders nach außen vor, sodass sie dort über eine elektrische Leitung mit der Versorgungseinheit verbunden werden kann. Bei unterschiedlicher Polung der Elektroden bilden sich an der Oberfläche des dielektrischen Materials bei ausreichender Feldstärke tangentiale Feldlinien aus, und es entsteht Plasma. Anstelle von zwei Bohrungen können auch mehrere Längsbohrungen im Zylinder und entsprechend eine höhere Anzahl von Elektroden vorhanden sein. Anordnung und Spannungsversorgung werden vorzugsweise so gewählt, dass sich tangentiale, aber keine radialen Felder bilden.

## Patentansprüche

1. Vorrichtung (1) zur Haut- und insbesondere Wundbehandlung unter Verwendung eines Atmosphärendruck-Plasmas, welche einen Plasmagenerator (2) aufweist, der wenigstens eine Elektrode (20, 20'), wenigstens ein Dielektrikum (21, 21'), eine elektrische Versorgungseinheit (22) sowie eine Steuereinheit (23) umfasst und ausgebildet ist, das Atmosphärendruck-Plasma mittels einer dielektrischen Barriereentladung zu erzeugen,
**dadurch gekennzeichnet,**
**dass** die Steuereinheit (23) ausgebildet ist, den Plasmagenerator (2) in unterschiedlichen Betriebsmodi derart zu betreiben, dass der wenigstens einen Elektrode (20, 20') in einem ersten Betriebsmodus (M1) eine erste Anregungsenergie zugeführt wird, die ausreicht, ein Stickstoffmonoxid (NO)-haltiges Plasma zu erzeugen, und in einem zweiten Betriebsmodus (M2) eine zweite Anregungsenergie, die ausreicht, ein Ozon (O₃)-haltiges Plasma zu erzeugen, wobei die erste Anregungsenergie größer ist als die zweite Anregungsenergie.

2. Vorrichtung nach Anspruch 1,
**dadurch gekennzeichnet,**
**dass** sie eine Benutzerschnittstelle (3) aufweist, die einem Benutzer die Auswahl zwischen den unterschiedlichen Betriebsmodi (M1, M2) erlaubt.

3. Vorrichtung nach Anspruch 1 oder 2,
**dadurch gekennzeichnet,**
**dass** sie eine Plasmakammer (4) zur Erzeugung des Plasmas und optional wenigstens eines der folgenden Merkmale aufweist: eine Gasversorgung (5) zur Zufuhr von Gas in die Plasmakammer (4), eine aus der Plasmakammer (4) herausgeführte Leitung (6) zur Zufuhr des erzeugten Plasmas zu einer zu behandelnden Wunde, eine Gastransportvorrichtung (7), insbesondere einen Ventilator oder eine Pumpe, eine vorzugsweise selbstklebende Folie zur Abdeckung der Wunde und/oder zur Befestigung zumindest eines Teils der Vorrichtung auf der Haut.

4. Vorrichtung nach einem der vorhergehenden Ansprüche,
**dadurch gekennzeichnet,**
**dass** sie einen mit Flüssigkeit (F), bevorzugt einer wässrigen Flüssigkeit, insbesondere gereinigtem Wasser, einer physiologischen Salzlösung oder einer Pufferlösung, befüllten Behälter (B) umfasst, in den eine aus der Plasmakammer (4) herausgeführte Leitung (6) zur Einleitung des gebildeten Plasmagases (P) in die Flüssigkeit einmündet.

5. Vorrichtung nach einem der vorhergehenden Ansprüche,
**dadurch gekennzeichnet,**
**dass** erste und zweite Anregungsenergie als Plasmaleistung in Bezug auf die Entladungsfläche bemessen sind, wobei die erste Anregungsenergie mehr als 0,25 W/cm², bevorzugt mehr als 0,4 W/cm², beträgt und die zweite Anregungsenergie im Bereich von 0,01 bis 0,25 W/cm², bevorzugt 0,01 bis 0,1 W/cm², liegt.

6. Vorrichtung nach einem der vorhergehenden Ansprüche,
**dadurch gekennzeichnet,**
**dass** die Versorgungseinheit (22) ausgebildet ist, der wenigstens einen Elektrode (20, 20') die Anregungsenergie als gepulste Spannung zuzuführen, wobei die zweite Anregungsenergie bevorzugt in Form von Pulsgruppen zugeführt wird, in denen mehrere Pulse zusammengefasst sind, wobei die Einzelpulse kürzer als 1 *µ*s sind.

7. Verfahren zur Behandlung der Haut und insbesondere einer Wunde unter Verwendung der Vorrichtung (1) nach einem der vorhergehenden Ansprüche,
**gekennzeichnet durch** die folgenden Schritte:
- Auswählen eines der Betriebsmodi aus dem ersten und dem zweiten Betriebsmodus,
- Steuern der Versorgungseinheit (22) durch die Steuereinheit (23) entsprechend dem ausgewählten Betriebsmodus (M1, M2), sodass der wenigstens einen Elektrode (20, 20') die erste Anregungsenergie oder die zweite Anregungsenergie zugeführt wird,
und optional wenigstens einen der weiteren Schritte:
- Einleiten von Gas in eine Plasmakammer (4) mittels einer Gastransportvorrichtung (7),
- Zuführen eines Plasmagases (P) aus der Plasmakammer (4) über eine Leitung (6) zum Behandlungsort oder
- Zuführen eines Plasmagases (P) aus der Plasmakammer (4) in einen mit einer Flüssigkeit (F), insbesondere einer wässrigen Flüssigkeit, befüllten Behälter (B).

8. Verfahren nach Anspruch 7,
**dadurch gekennzeichnet,**
**dass** als Gas eine Sauerstoff und Stickstoff enthaltende Gasmischung und insbesondere Luft verwendet wird, wobei die Gasmischung bevorzugt wenigstens eine der folgenden Eigenschaften aufweist:
- eine Temperatur von 15 bis 60 °C,
- eine relative Feuchte von 20 bis 90 %,
- einen Schwebstoffgehalt von 0,1 bis 20 mg/m³,
- einen Durchfluss von 0,5 bis 10 slm.

9. Verfahren nach Anspruch 7 oder 8,
**dadurch gekennzeichnet,**
**dass** die Versorgungseinheit (22) bei Auswahl des ersten Betriebsmodus (M1) eine erste Anregungsenergie mit mehr als 0,25 W/cm², bevorzugt mehr als 0,4 W/cm², und bei Auswahl des zweiten Betriebsmodus (M2) eine zweite Anregungsenergie im Bereich von 0,01 bis 0,25 W/cm², bevorzugt 0,01 bis 0,1 W/cm², jeweils angegeben als Plasmaleistung in Bezug auf die Entladungsfläche, zuführt.

10. Verfahren nach einem der Ansprüche 7 bis 9,
**dadurch gekennzeichnet,**
**dass** die Versorgungseinheit (22) der wenigstens einen Elektrode (20, 20') die Anregungsenergie als gepulste Spannung zuführt, wobei sie die zweite Anregungsenergie bevorzugt in Form von Pulsgruppen zuführt, in denen mehrere Pulse zusammengefasst sind, wobei die Einzelpulse kürzer als 1 *µ*s sind.

11. Verfahren nach einem der Ansprüche 7 bis 10,
**dadurch gekennzeichnet,**
**dass** die Barrierespannung für den ersten Betriebsmodus (M1) zwischen 2000 und 4000 V, insbesondere bei 2500 bis 3500 V, und/oder für den zweiten Betriebsmodus (M2) zwischen 900 und 2000 V, insbesondere bei 1000 bis 1700 V, liegt.
